# EUROPÄISCHE PATENTSCHRIFT

(11) **EP 0 946 424 B1**
(45) Veröffentlichungstag und Bekanntmachung des Hinweises auf die Patenterteilung: **15.09.2004**
(21) Anmeldenummer: 97953877.4
(22) Anmeldetag: 19.12.1997
(51) Int. Cl.: C02F 1/32, A61L 2/10

(54) **STRÖMUNGSGÜNSTIGE UV-DESINFEKTIONSVORRICHTUNG**
UV DISINFECTING DEVICE ADVANTAGEOUS FOR FLUID FLOWS
DISPOSITIF DE DESINFECTION PAR U.V. FAVORISANT L'ECOULEMENT

(30) Priorität: 19.12.1996 DE 19653083
(43) Veröffentlichungstag der Anmeldung: 06.10.1999
(73) Patentinhaber: Wedeco AG, 40472 Düsseldorf (DE)
(72) Erfinder: WEDEKAMP, Horst, D-32052 Herford (DE)
(74) Vertreter: Lenzing, Andreas, Dr.
(86) Internationale Anmeldenummer: PCT/EP1997/007174
(87) Internationale Veröffentlichungsnummer: WO 1998/027011

(56) Entgegenhaltungen:
- EP-A- 0 080 780
- DE-C- 250 841
- GB-A- 2 030 694
- US-A- 3 061 721
- US-A- 4 367 410
- US-A- 4 767 932
- US-A- 4 825 083
- US-A- 5 124 131

## Beschreibung

Die vorliegende Erfindung betrifft eine UV-Desinfektionsvorrichtung für strömende Fluide mit den Merkmalen des Oberbegriffs des Anspruchs 1.

Derartige Vorrichtungen werden in den aus einer Kläranlage austretenden, geklärten Abwasserstrom eingebaut. Dieser Abwasserstrom wird durch einen offenen Kanal geleitet, in den die gattungsgemäßen UV-Desinfektionseinrichtungen eingesetzt sind. Dabei sind langgestreckte UV-Strahler in Form von Gasentladungslampen im wesentlichen horizontal und mit ihrer Längsachse in Strömungsrichtung angeordnet. Je nach Abwassermenge werden mehr oder weniger Strahler parallel nebeneinander und übereinander eingesetzt, so daß jeder Punkt des Abwasserstroms einen bestimmten Maximalabstand von den Strahlungsquellen nicht überschreitet. Auf diese Weise werden die im geklärten Abwasser befindlichen Bakterien zuverlässig abgetötet und das Abwasser auf diese Weise desinfiziert.

Eine gattungsgemäße UV-Desinfektionsvorrichtung ist aus der EP 0080780 bekannt. Bei dieser Ausführungsform sind mehrere UV-Lampen von Hüllrohren umgeben und bilden mit diesen gemeinsam Lampeneinheiten. Mehrere Lampeneinheiten sind horizontal, parallel zueinander und übereinander angeordnet, wobei die Hüllrohre jeweils wasserdicht in rahmenfesten Sockeln angeordnet sind. Das Innere der Hüllrohre, das Innere der Sockel und das Rahmeninnere stehen auf diese Weise miteinander in Verbindung, so daß die elektrischen Zuleitungen durch diese Hohlräume geführt werden können.

Problematisch ist bei dieser Ausführungsform, daß zum einen diese Anlage technisch aufwendig ist. Zum zweiten ist zum Auswechseln einer einzigen defekt gewordenen UV-Lampe das wasserdicht abgeschlossene System zu öffnen, was insbesondere bei äußerer Verschmutzung später zu Dichtigkeitsproblemen führen kann. Schließlich besteht die nicht unerhebliche Gefahr, daß bei einer einzigen Undichtigkeit im gesamten System sämtliche miteinander kommunizierenden Hohlräume mit Abwasser geflutet werden und hierdurch eine ganze senkrechte Reihe von UV-Strahlern ausfällt und schwer beschadigt wird.

Daher ist es Aufgabe der vorliegenden Erfindung, eine UV-Desinfektionsvorrichtung für strömende Fluide zu schaffen, die konstruktiv einfacher, leichter zu warten und zuverlässiger im Betrieb ist.

Diese Aufgabe wird von einer Vorrichtung mit den Merkmalen des Anspruchs 1 gelost.

Weil zur Halterung der Lampeneinheiten Klammern vorgesehen sind, können einzelne Lampeneinheiten einfach aus der Desinfektionsvorrichtung entnommen werden, ohne die sichere und wasserdichte Montage der übrigen Lampeneinheiten zu beeinträchtigen. Die Klammern sind preiswert zu fertigen und bieten darüber hinaus den Vorteil, daß der in Richtung der Langsachse der einzelnen Lampeneinheiten laufenden Strömung des zu desinfizierenden Abwassers ein geringer Strömungswiderstand entgegengesetzt wird.

Eine gute Standzeit der Halterung ergibt sich, wenn die Klammern aus einem korrosionsbeständigen Federstahl gefertigt sind, beispielsweise als Stanzbiegeteile aus einem Bandstahl.

Eine zuverlässige und elektrisch besonders sichere Konfiguration wird erreicht, wenn die UV-Lampen von einem Hüllrohr umgeben ist, das für UV-Strahlung auch durchlässig ist. Eine sichere Halterung auch bei mäßigen Belastungen der Lampeneinheiten ergibt sich, wenn die Klammern eine derartige Öffnungsweite aufweisen, daß sie die Lampeneinheiten in deren Umfangsbereich mit oder ohne zwischengefügte Bauelemente kraftschlüssig halten. Dabei können den Lampeneinheiten zumindest zum Teil Halteringe zugeordnet sein, die sich im Betrieb zwischen den Hüllrohren und den Klammern befinden. Diese Ausführungsform ist besonders sicher gegen Beschadigungen der Hüllrohre.

In besonders einfacher Weise können die Hüllrohre dadurch wasserdicht verschlossen werden, daß an wenigstens einem Ende mit einem Stopfen aus gummielastischem und/oder UV-beständigem Material verschlossen sind. Auf diese Weise erübrigt sich eine komplexe Verschlußvorrichtung, die zahlreiche Dichtflächen und damit Fehlerquellen aufweist. Insbesondere ist vorteilhaft, wenn der Stopfen an seinem äußeren Umfang in dem in die Hüllrohre einführbaren Bereich mit umlaufenden Rippen versehen ist, die im Betrieb dichtend an der inneren Oberfläche der Hüllrohre anliegen. Derartige Rippen dichten in derselben Weise, wie beispielsweise O-Ringe an dieser Stelle dichten würden. Dabei kann der wenigstens eine elektrische Anschluß durch den Stopfen geführt sein. Der elektrische Anschluß kann eingegossen oder vulkanisiert in dem Stopfen gehalten sein.

Es ist beispielsweise für die Sicherung der Position des Stopfens einer Lampeneinheit vorteilhaft, wenn bei jeder Lampeneinheit eine oder zwei Klammern unmittelbar den jeweiligen Stopfen ergreifen und nicht das entsprechende Ende des Hüllrohres.

Weiter ist es vorteilhaft, wenn zur Reinigung der äußeren Oberfläche ein Wischer vorgesehen ist, der die Hüllrohre umgebende Reinigungselemente in Axialrichtung des jeweiligen Hüllrohres schieben kann und die Reinigungselemente, beispielsweise in Form von Abstreifringen, an dem Wischer mittels Klammern gehalten sind, die vorzugsweise baugleich mit den Klammern zur Halterung der Lampeneinheiten sind. Auf diese Weise wird ein Herausnehmen der Lampeneinheit sowohl aus dem Rahmen als auch aus dem Wischer ermöglicht, wobei identische Bauteile zum Einsatz kommen. Wenn wenigstens ein Stopfen dabei den Außendurchmesser des Hüllrohres nicht überragt, kann der jeweilige Abstreifring zur Wartung von dem Hüllrohr abgezogen werden, ohne die Lampeneinheit zu öffnen.

Wenn schließlich die Hüllrohre jeweils wenigstens eine umlaufende Einschnürung oder Nut aufweisen, in deren Bereich die Lampeneinheiten von den Klammern gehalten sind. Dann wird die jeweilige Lampeneinheit in Axialrichtung fest fixiert und bleibt in einer Radialrichtung einfach und ohne Werkzeug entnehmbar. Der Strömungswiderstand wird durch diese Maßnahme weiter reduziert.

Ein Ausführungsbeispiel der vorliegenden Erfindung ist im folgenden anhand der Zeichnung beschrieben.

Es zeigen:
- Figur 1:: Ein erfindungsgemäßes Modul einer Desinfektionsvorrichtung in einer verkürzten Seitenansicht;
- Figur 2:: das Modul gemaß Figur 1 in einer Stirnansicht;
- Figur 3:: ein mit einem Stopfen verschlossenes Ende einer Lampeneinheit in einem Querschnitt von der Seite;
- Figur 4:: ein mit einem Stopfen verschlossenes Ende einer Lampeneinheit in einem Querschnitt von der Seite, wobei der Stopfen das Hüllrohr im Durchmesser nicht überragt; sowie
- Figur 5:: ein Ende einer Lampeneinheit mit einer randseitigen Nut.

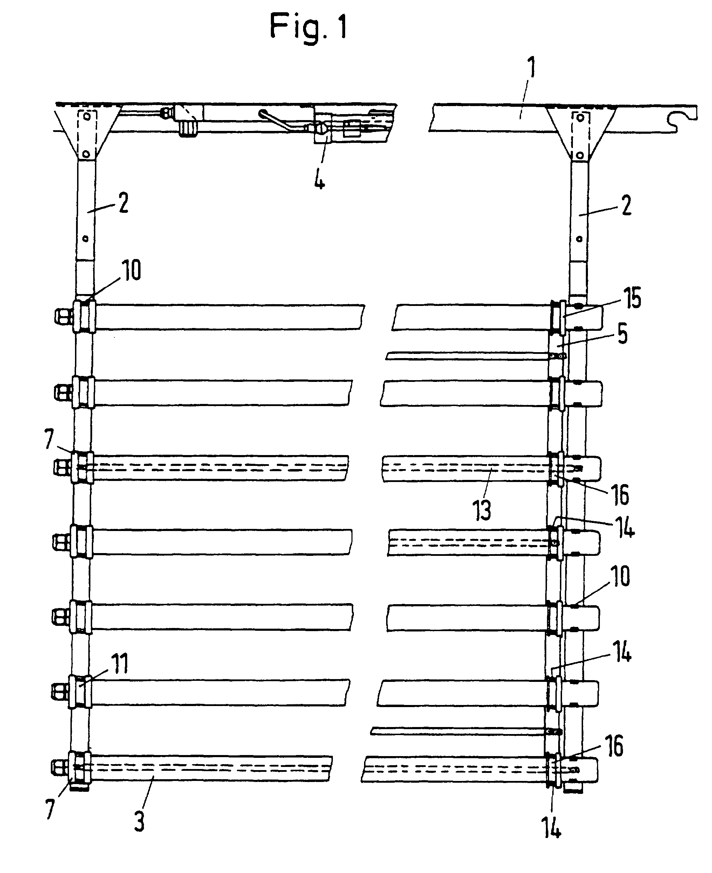

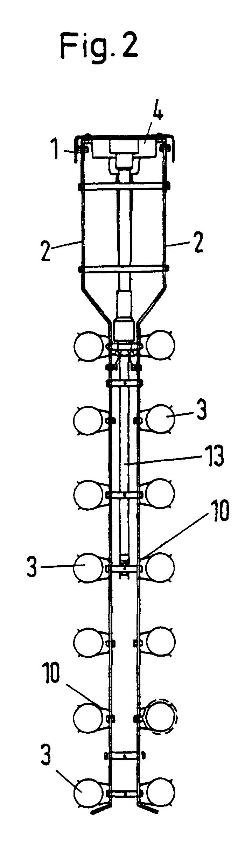

In der Figur 1 und der Figur 2 ist ein Modul einer UV-Desinfektionsvorrichtung in einer verkürzten Seitenansicht sowie in einer Stirnansicht dargestellt.

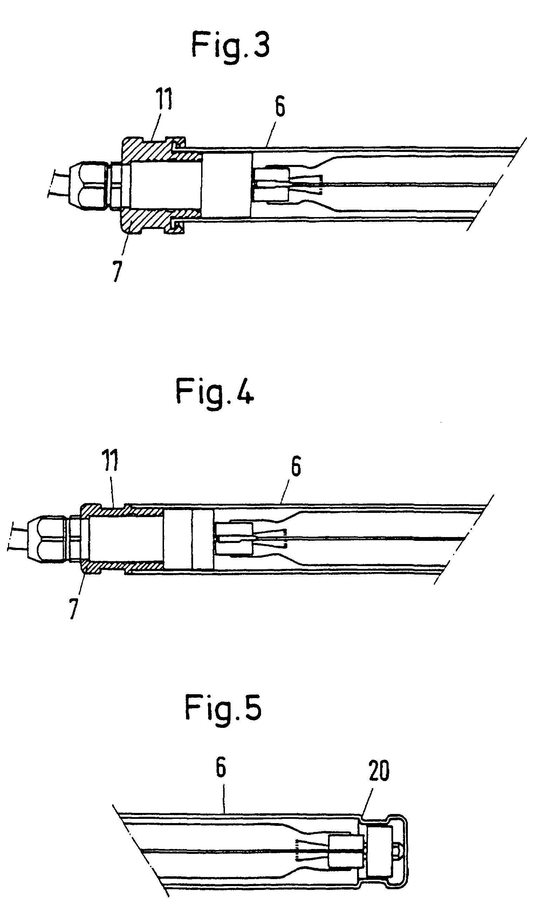

Das Modul umfaßt einen im Betrieb horizontal verlaufenden Rahmenträger 1, von dem je zwei senkrechte Rahmenschenkel 2 in senkrechter Richtung nach unten wegstehen. Die Rahmenschenkel 2 wiederum tragen insgesamt 14 Lampeneinheiten 3, von denen in der Figur 1 nur sieben sichtbar sind. Dem horizontalen Rahmenträger 1 sind nur zum Teil abgebildete Antriebsmittel 4 für eine Wischereinheit 5 zugeordnet. Die Lampeneinheiten 3 haben im wesentlichen längliche zylindrische Form, deren äußere Gestalt von Hüllrohren 6 und je einem Verschlußstopfen 7 bestimmt ist, wie aus den Figuren 3 und 4 ersichtlich ist. An ihre dem Verschlußstopfen 7 gegenüberliegenden Ende sind die Lampeneinheiten in einem geschlossenen Ende einstückig abgeschlossen.

Die Lampeneinheiten 3 sind an den senkrecht verlaufenden Rahmenschenkeln 2 mit Klammern 10 gehalten, die die jeweilige Lampeneinheit an beiden Enden kraftschlüssig ergreifen. Die jeweilige Klammer 10 erfaßt das stopfenseitige Ende der Lampeneinheit im Bereich einer um dem Stopfen umlaufenden Nut 11, während das dem Stopfen gegenüberliegende Ende unmittelbar im Bereich der Oberfläche des Hüllrohres 6 ergriffen wird. Die Lampeneinheit ist auf diese Weise in den Klammern 10 in einer eindeutigen Position fixiert, denn an beiden Enden bewirken die Klammern 10 eine kraftschlüssige Festlegung in Radialrichtung, während der Eingriff in die Nut 11 im Bereich des Stopfens eine formschlüssige Festlegung in Axialrichtung bewirkt. Die Wischereinheit 5 hat einen pneumatischen Linearzylinder als Antrieb im Bereich der Antriebseinheit 4 sowie eine Strebenanordnung 13, die endseitig jeweils jeder Lampeneinheit benachbart eine Klammer 14 trägt. Die Klammern 14 sind baugleich mit den Klammern 10. Jede Klammer 14 wiederum greift in einen das entsprechende Hüllrohr 6 umgebenden Ring 15 ein, in dem die Klammer 14 im Bereich einer Ringnut 16 zumindest in Axialrichtung formschlüssig sitzt. Der Ring 15 umgibt das jeweilige Hüllrohr 3 einer Lampeneinheit in einem engen Gleitsitz.

Die Figur 4 zeigt ein mit einem Stopfen verschlossenes Ende einer Lampeneinheit in einem Querschnitt von der Seite, wobei der Stopfen 7 das Hüllrohr 6 im Durchmesser nicht überragt. So wird erreicht, daß der Abstreifring 14 der Wischereinheit 5 auch über die Anschlußseite der Lampeneinheit abgenommen werden kann, ohne daß diese geöffnet werden muß. Der umströmte Querschnitt der Lampeneinheit wird so besonders klein.

Figur 5 zeigt schließlich ein Ende einer Lampeneinheit, bei dem eine ringförmig umlaufende Nut oder Einschnürung 20 von der Außenseite her eingeprägt ist. Eine Klammer greift in der Betriebsstellung in diese Nut ein. Dadurch wird die Lampeneinheit in ihrer Längsrichtung formschlüssig fixiert, während sie in Richtung der offenen Seite der Klammer leicht entnehmbar bleibt. Das Eingreifen der Klammer in die Nut 20 verringert den Strömungswiderstand. Außerdem ist es nicht länger erforderlich, eine Fixierung in Längsrichtung im Bereich der Stopfen 7,11 vorzusehen. Dort kann die Klammer in einem glatten Bereich des Stopfens oder des Hüllrohres angreifen. Eine zweite Fixierung in der Längs- oder Axialrichtung der Lampeneinheiten ist im allgemeinen nicht erforderlich.

Im Betrieb wird das Modul der dargestellten UV-Desinfektionsanlage in einen Kanal, ein sogenanntes Gerinne, einer Kläranlage eingesetzt und emittiert dort Ultraviolettstrahlung hoher Energiedichte, während das zu desinfizierende Abwasser die Lampeneinheiten in horizontaler Richtung parallel zur Achsrichtung der Hüllrohre 6 umströmt. Je nach Zusammensetzung des Wassers entstehen früher oder später Ablagerungen auf den äußeren Oberflächen der Hüllrohre, die die Intensität der ultravioletten Strahlung verringern. Deshalb wird in regelmäßigen Abständen das Gestänge 13 der Wischereinheit 5 durch Aktivierung des Antriebs 4 parallel zur Achsrichtung der Hüllrohre 6 verschoben. Hierbei nehmen die endseitigen Klammern 14 die Ringe 15 mit und wischen auf diese Weise nahezu die gesamte Oberfläche der Hüllrohre 6 frei.

Falls eine Lampe wegen Defekt oder nachlassender Leistung ausgetauscht oder das Modul in sonstiger Weise gewartet werden muß, kann das Bedienpersonal das entsprechende Modul einfach aus dem Gerinne herausheben und die zu bearbeitende Lampeneinheit durch Handkraft quer zur Achsrichtung der Hüllrohre 6 aus den Klammern herausziehen. Hierbei sind lediglich die Haltekräfte der Klammern 10 bzw. 14 zu überwinden, ohne daß Werkzeug erforderlich wäre. Gleichzeitig wird der Ring 15 zusammen mit der Lampeneinheit aus der Wischeranordnung 5, 13, 14 herausgelöst und kann ebenfalls gereinigt oder ersetzt werden. Der Stopfen 7 ist bei separat vorliegender Lampeneinheit aus dem Hüllrohr 6 in einfacher Weise zu entfernen. Da der Stopfen 7 aus einem gummielastischen Material gefertigt ist, kann er einfach aus dem Hüllrohr 6 herausgezogen werden, wobei auch die in dem Hüllrohr 6 befindliche UV-Lampe zugänglich wird. Das Zusammensetzen der Lampeneinheit erfolgt dann in umgekehrter Weise, d. h. die UV-Lampe wird in den dem Stopfen 7 zugeordneten Sockel eingesetzt und dann gemeinsam mit der UV-Lampe in das Hüllrohr 7 eingeführt. Dabei werden an dem Stopfen 7 angeformte, ringförmig umlaufende Rippen, die an der Innenseite des Hüllrohres 6 dichtend anliegen, während die Stirnseite des Hüllrohres 6 in eine Nut des Stopfens 7 eingeführt und dort von einem Hinterschnitt gehalten werden. Die Dichtflächen des Stopfens 7 mit dem Hüllrohr 6 sind von außen durch das transparente Hüllrohr hindurch problemlos zu sehen, so daß in der Praxis eine unzuverlässig einliegende Dichtung beispielsweise in Folge von Verschmutzung unmittelbar erkannt werden kann.

Bei der vorliegenden Ausführungsform sind also nicht nur sämtliche Lampeneinheiten separat aus dem jeweiligen Modul zu entfernen. Die Halterung der jeweiligen Lampeneinheiten in Klammern ist außerdem eine ausgesprochen einfache und kostengünstige, aber sehr zuverlässige Halterungsmöglichkeit. Diese Halterung setzt auch dem in Axialrichtung strömenden Wasser nur geringen Widerstand entgegen, so daß auch bei großen Durchflußmengen nur ein geringer Druckverlust entsteht. Die relativ einfache Abdichtung der Lampeneinheiten mit dem beschriebenen Stopfen 7 erhöht darüber hinaus die Zuverlässigkeit der Module, indem auch im unwahrscheinlichen Falle einer mangelhaften Abdichtung lediglich eine Lampeneinheit ausfallen würde und nicht gleich das gesamte Modul mit allen Lampeneinheiten beschädigt wird.

## Patentansprüche

1. UV-Desinfektionsvorrichtung für strömende Fluide, mit einem Rahmen (1) sowie mit einer Anzahl von UV-Lampen aufweisenden Lampeneinheiten (3), die über jeweils wenigstens einen elektrischen Anschluß verfügen und die von dem Rahmen (1) im wesentlichen parallel und im Abstand voneinander gehalten sind,
wobei jede Lampeneinheit (3) ein die UV-Lampe umgebendes Hüllrohr (6) aufweist, **dadurch gekennzeichnet, daß** die Hüllrohre (6) un wenigstens einem Ende mit einem Stopfen (7) aus gummielastischem und/oder UV-beständigem Material verschlossen sind und daß zur Halterung der Lampeneinheiten (3) Klammern (10) vorgesehen sind.

2. Vorrichtung nach Anspruch 1, **dadurch gekennzeichnet, daß** die Klammern (10) aus einem korrosionsbeständigen Federstahl gefertigt sind.

3. Vorrichtung nach einem der vorhergehenden Ansprüche, **dadurch gekennzeichnet, daß** die Klammern (10) eine derartige Öffnungsweite aufweisen, daß sie die Lampeneinheiten (3) in deren Umfangsbereich kraftschlüssig halten.

4. Vorrichtung nach einem der vorhergehenden Ansprüche, **dadurch gekennzeichnet, daß** den Lampeneinheiten (3) zumindest zum Teil Ringe (15) zugeordnet sind, die sich im Betrieb zwischen den Hüllrohren (6) und den Klammern (10) befinden und die als Abstreifringe oder als Halteringe für Abstreifer ausgebildet sind.

5. Vorrichtung nach einem der vorhergehenden Ansprüche, **dadurch gekennzeichnet, daß** der Stopfen (7) an seinem äußeren Umfang in dem in die Hüllrohre (6) einführbaren Bereich mit umlaufenden Rippen versehen ist, die im Betrieb dichtend an der inneren Oberfläche der Hüllrohre (6) anliegen.

6. Vorrichtung nach einem der vorhergehenden Ansprüche, **dadurch gekennzeichnet, daß** der Stopfen (7) eine in Axialrichtung offene, umlaufende Nut (11) aufweist, in die die Stirnseite des Hüllrohres (6) einsetzbar ist.

7. Vorrichtung nach einem der vorhergehenden Ansprüche, **dadurch gekennzeichnet, daß** der wenigstens eine elektrische Anschluß durch den Stopfen (7) geführt ist.

8. Vorrichtung nach einem der vorhergehenden Ansprüche, **dadurch gekennzeichnet, daß** die Lampeneinheiten (3) zum Teil von Klammern (10) gehalten sind, die unmittelbar den jeweiligen Stopfen (7) ergreifen.

9. Vorrichtung nach einem der vorhergehenden Ansprüche, **dadurch gekennzeichnet, daß** zur Reinigung der äußeren Oberfläche der Hüllrohre eine Wischereinheit (5) vorgesehen ist, der die Hüllrohre (6) umgebende Reinigungselemente (5,13,14) in Axialrichtung des jeweiligen Hüllrohres (6) verschieben kann, wobei die Reinigungselemente (5,13,14) an der Wischereinheit (5) mittels Klammern (14) gehalten sind, die vorzugsweise baugleich mit den Klammern (10) zur Halterung der Lampeneinheiten (3) sind.

10. Vorrichtung nach einem der vorhergehenden Ansprüche, **dadurch gekennzeichnet, daß** die Hüllrohre jeweils wenigstens eine umlaufende Einschnürung oder Nut (20) aufweisen.

11. Vorrichtung nach einem der vorhergehenden Ansprüche, **dadurch gekennzeichnet, daß** die Lampeneinheiten (3) im Bereich der Nut (20) von Klammern (10,14) gehalten sind, die unmittelbar die Lampeneinheiten ergreifen und in deren Axialrichtung fixieren.

## Claims

1. UV disinfecting device for flowing fluids, having a frame (1) and having a number of lamp units (3) having UV lamps, which lamp units (3) each have at least one electrical terminal and are held by the frame (1) substantially parallel and at a distance from one another, each lamp unit (3) having an outer tube (6) which surrounds the UV lamp, **characterised in that** the outer tubes (6) are closed off at at least one end by a plug (7) of elastomeric and/or UV-resistant material and **in that** clips (10) are provided to act as mountings for the lamp units (3).

2. Device according to claim 1, **characterised in that** the clips (10) are made of a corrosion-resistant spring steel.

3. Device according to one of the foregoing claims, **characterised in that** the diameter of the clips (10) is such that they hold the lamp units (3) by frictional engagement in the circumferential region of the latter.

4. Device according to one of the foregoing claims, **characterised in that** at least some of the lamp units (3) have associated with them rings (15) which, in operation, are situated between the outer tubes (6) and the clips (10) and which are in the form of cleaner rings or mounting rings for cleaners.

5. Device according to one of the foregoing claims, **characterised in that**, in the region that is insertable in the outer tube (6), the plug (7) is provided on its outer circumference with circumferential ribs which, in operation, bear against the inside surface of the outer tube (6) with a seal.

6. Device according to one of the foregoing claims, **characterised in that** the plug (7) has a circumferential groove (11) which is open in the axial direction and into which the end of the outer tube (6) can be inserted.

7. Device according to one of the foregoing claims, **characterised in that** the at least one electrical terminal is run through the plug (7).

8. Device according to one of the foregoing claims, **characterised in that** some of the lamp units (3) are held by clips (10) which directly grasp the respective plugs (7).

9. Device according to one of the foregoing claims, **characterised in that** there is provided, for cleaning the outer surface of the outer tube, a wiper unit (5) which is able to move cleaning elements (5, 13, 14) surrounding the outer tube (6) in the axial direction of the particular outer tube (6), the cleaning elements (5, 13, 14) being held on the wiper unit (5) by means of clips (14) which are preferably of the same design as the clips (10) acting as mountings for the lamp units (3).

10. Device according to one of the foregoing claims, **characterised in that** the outer tubes each have at least one circumferential constriction or groove (20).

11. Device according to one of the foregoing claims, **characterised in that** the lamp units (3) are held in the region of the groove (20) by clips (10, 14) which grasp the lamp units directly and locate the lamp units in the axial direction of the latter.

## Revendications

1. Dispositif de désinfection par ultraviolet pour des liquides en circulation, comprenant un cadre (1), ainsi que des unités de lampes (3), qui sont munies d'un nombre donné de lampes UV et dont chacune dispose d'au moins un connecteur électrique et sont maintenues sensiblement parallèlement à distance du cadre (1) et les unes des autres, chaque unité de lampe (3) comportant un tube de protection (6) entourant la lampe UV, **caractérisé en ce que** les tubes de protection (6) sont fermés au niveau d'au moins une extrémité par un bouchon (7) réalisé dans un matériau élastique de type caoutchouc et/ou résistant au rayonnement ultraviolet et **en ce qu'**il est prévu des brides (10) pour maintenir les unités de lampes (3).

2. Dispositif selon la revendication 1, **caractérisé en ce que** les brides (10) sont réalisées dans un acier pour ressorts résistant à la corrosion.

3. Dispositif selon l'une quelconque des revendications précédentes, **caractérisé en ce que** les brides (10) ont une largeur d'ouverture telle qu'elles maintiennent les unités de lampes (3) par application de force dans la zone de leur pourtour.

4. Dispositif selon l'une quelconque des revendications précédentes, **caractérisé en ce que** des anneaux (15), qui sont disposés en cours de service entre les tubes de protection (6) et les brides (10) et qui sont réalisés sous forme d'anneaux racleurs ou d'anneaux de retenue pour racleurs, sont associés au moins en partie aux unités de lampe (3).

5. Dispositif selon l'une quelconque des revendications précédentes, **caractérisé en ce que** le bouchon (7), sur sa périphérie extérieure dans la zoné à introduire dans le tube de protection (6), est muni de nervures périphériques qui, en cours de service, sont en appui étanche contre la surface intérieure des tubes de protection (6).

6. Dispositif selon l'une quelconque des revendications précédentes, **caractérisé en ce que** le bouchon (7) comporte une rainure (11) périphérique ouverte dans le sens axial, destinée à recevoir la face frontale du tube de protection (6).

7. Dispositif selon l'une quelconque des revendications précédentes, **caractérisé en ce que** ledit au moins un connecteur électrique est guidé à travers le bouchon (7).

8. Dispositif selon l'une quelconque des revendications précédentes, **caractérisé en ce que** les unités de lampes (3) sont maintenues en partie par des brides (10) qui saisissent directement le bouchon (7) correspondant.

9. Dispositif selon l'une quelconque des revendications précédentes, **caractérisé en ce que**, pour le nettoyage de la surface extérieure des tubes de protection, il est prévu un dispositif d'essuyage (5), par lequel les éléments de nettoyage (5, 13, 14) entourant les tubes de protection (6) peuvent être déplacés dans le sens axial de chaque tube de protection (6), les éléments de nettoyage (5, 13, 14) étant maintenus contre le dispositif d'essuyage (5) au moyen de brides (14) qui, de préférence, sont conçues de manière identique aux brides (10) destinées à maintenir les unités de lampes (3).

10. Dispositif selon l'une quelconque des revendications précédentes, **caractérisé en ce que** les tubes de protection comportent chacun au moins un rétrécissement ou rainure (20) périphérique.

11. Dispositif selon l'une quelconque des revendications prêcédentés, **caractérisé en ce que** les unités de lampes (3) sont maintenues dans la zone de la rainure (20) par des brides (10, 14) qui saisissent directement les unités de lampes et les bloquent dans leur sens axial.
